# EUROPEAN PATENT APPLICATION

(11) **EP 3 489 683 A1**
(43) Date of publication of application: **29.05.2019**
(21) Application number: 17830648.6
(22) Date of filing: 23.03.2017
(51) Int. Cl.: G01N 33/53, G01N 1/04

(54) **TARGET ANALYSIS KIT AND TARGET ANALYSIS METHOD**

(30) Priority: 20.07.2016 JP 2016142546
(71) Applicant: NEC Solution Innovators, Ltd., Tokyo 136-8627 (JP)
(72) Inventor: YOSHIDA, Yoshihito, Tokyo 136-8627 (JP); HORII, Katsunori, Tokyo 136-8627 (JP); WAGA, Iwao, Tokyo 136-8627 (JP); AKITOMI, Jou, Tokyo 136-8627 (JP); KANEKO, Naoto, Tokyo 136-8627 (JP)
(74) Representative: Vossius & Partner Patentanwälte Rechtsanwälte mbB
(86) International application number: PCT/JP2017/011617
(87) International publication number: WO 2018/016127

(57) **Abstract**

The present invention provides a target analysis kit and a target analysis method that allow easily analysis of a target. A target analysis kit includes: a sample holder; and an analysis container. The sample holder includes: a reagent chamber containing a first reagent solution that is a solution containing a first reagent; a sample holding portion; and a liquid transfer portion that allows transfer of the first reagent solution in the reagent chamber to the holding portion. The analysis container includes: a first chamber; and a second chamber, The first chamber and the second chamber are disposed continuously in this order. The first chamber contains a second reagent. The second chamber is a detection portion in which a labeling substance contained in the first reagent or the second reagent is to be detected. A porous partition wall is provided between the first chamber and the second chamber. The first chamber is configured such that the sample holder holding a sample can be inserted from an outside of the first chamber to an inside of the first chamber. The porous partition wall is a porous partition wall through which an immobilized first binding substance obtained by immobilizing on a carrier a first binding substance that binds to a target and contained in the first reagent or the second reagent or an immobilized second binding substance obtained by immobilizing on a carrier a second binding substance that binds to the first binding substance and contained in the first reagent or the second reagent cannot pass and a conjugate composed of a labeled first binding substance obtained by binding a labeling substance to the first binding substance and the target and contained in the first reagent or the second reagent or a labeled second binding substance obtained by binding a labeling substance to the second binding substance and contained in the first reagent or the second reagent can pass. A combination of the first reagent and the second reagent is any of the following combinations (1) to (3) and (4):
(1) the first reagent is the immobilized second binding substance and the second reagent is the labeled first binding substance;
(2) the first reagent is the labeled first binding substance and the second reagent is the immobilized second binding substance;
(3) the first reagent is the immobilized first binding substance and the second reagent is the labeled second binding substance; and
(4) the first reagent is the labeled second binding substance and the second reagent is the immobilized first binding substance.

## Description

### TECHNICAL FIELD

The present invention relates to a target analysis kit and a target analysis method.

### BACKGROUND ART

In general, target analysis is performed using binding substances having binding affinity to a target, and the presence or absence of the target or the amount of the target can be analyzed by causing the binding substances to form conjugates with the target and detecting the thus-formed conjugates directly or indirectly (Patent Documents 1 and 2).

However, an analysis method performed using binding substances in the above-described manner requires a process of separating the binding substances bound to the target and the binding substances not bound to the target. Accordingly, it is difficult to perform a series of processes and detect the target in a single analysis tool (analysis container).

### Citation List

### Patent Literature(s)

[Patent Literature 1] Japanese Translation of PCT International Application Publication No. JP-T-2014-507670
[Patent Literature 2] Japanese Translation of PCT International Application Publication No. JP-T-2007-518994

### SUMMARY OF INVENTION

### Technical Problem

With the foregoing in mind, it is an object of the present invention to provide a target analysis kit for easy analysis of a target.

### Solution to Problem

The present invention provides a target analysis kit (also referred to as "analysis kit" hereinafter) including: a sample holder; and an analysis container. The sample holder includes: a reagent chamber containing a first reagent solution that is a solution containing a first reagent; a sample holding portion; and a liquid transfer portion that allows transfer of the first reagent solution in the reagent chamber to the holding portion. The analysis container includes: a first chamber; and a second chamber, The first chamber and the second chamber are disposed continuously in this order. The first chamber contains a second reagent. The second chamber is a detection portion in which a labeling substance contained in the first reagent or the second reagent is to be detected. A porous partition wall is provided between the first chamber and the second chamber. The first chamber is configured such that the sample holder holding a sample can be inserted from an outside of the first chamber to an inside of the first chamber. The porous partition wall is a porous partition wall through which an immobilized first binding substance obtained by immobilizing on a carrier a first binding substance that binds to a target and contained in the first reagent or the second reagent or an immobilized second binding substance obtained by immobilizing on a carrier a second binding substance that binds to the first binding substance and contained in the first reagent or the second reagent cannot pass and a conjugate composed of a labeled first binding substance obtained by binding a labeling substance to the first binding substance and the target and contained in the first reagent or the second reagent or a labeled second binding substance obtained by binding a labeling substance to the second binding substance and contained in the first reagent or the second reagent can pass. A combination of the first reagent and the second reagent is any of the following combinations (1) to (3) and (4):
(1) the first reagent is the immobilized second binding substance and the second reagent is the labeled first binding substance;
(2) the first reagent is the labeled first binding substance and the second reagent is the immobilized second binding substance;
(3) the first reagent is the immobilized first binding substance and the second reagent is the labeled second binding substance; and
(4) the first reagent is the labeled second binding substance and the second reagent is the immobilized first binding substance.

The present invention also provides a target analysis method (also referred to as "analysis method" hereinafter) using the target analysis kit according to the present invention. The target analysis method includes the steps of: introducing a sample and the first reagent solution to the first chamber by inserting the sample holder holding the sample into the first chamber of the analysis container and then transferring the first reagent solution in the reagent chamber to the holding portion; in the first chamber, bringing the sample into contact with the first reagent and the second reagent to form a conjugate of the target in the sample and the first binding substance and also binding an unbound first binding substance not bound to the target to the second binding substance; introducing an unbound labeled second binding substance not bound to the immobilized first binding substance or an unbound labeled first binding substance not bound to the immobilized second binding substance to the second chamber through the porous partition wall between the first chamber and the second chamber; and detecting the labeling substance in the labeled first binding substance or the labeled second binding substance in the second chamber.

### Advantageous Effects of Invention

According to the target analysis kit of the present invention, a target in a sample can be analyzed easily.

### BRIEF DESCRIPTION OF DRAWINGS

FIG. 1 is a schematic view showing an example of a target analysis kit of a first example embodiment.
FIGs. 2A to 2C schematically show an example of an analysis method using the target analysis kit of the first example embodiment.
FIG. 3 is a schematic view showing an example of a target analysis kit of a second example embodiment.
FIGs. 4A to 4C schematically show an example of an analysis method using the target analysis kit of the second example embodiment.
FIG. 5 is a schematic view showing an example of a target analysis kit of a third example embodiment.
FIGs. 6A to 6C schematically show an example of an analysis method using the target analysis kit of the third example embodiment.

### DESCRIPTION OF EMBODIMENTS

The analysis kit of present invention may be configured such that, for example, the liquid transfer portion includes: a tube that connects the reagent chamber and the holding portion; and a sealing portion that seals the tube in the reagent chamber, and the first reagent solution in the reagent chamber is transferred to the holding portion when the liquid transfer portion is broken inside the reagent chamber by force applied from an outside of the reagent chamber, thereby opening the tube.

The analysis kit of present invention may be configured such that, for example, the liquid transfer portion includes a tube that connects the reagent chamber and the holding portion; and a lid disposed so as to block a connected portion between the reagent chamber and the tube, and the first reagent solution in the reagent chamber is transferred to the holding portion when the lid is moved by force applied from an outside of the reagent chamber, thereby opening the connected portion.

The analysis kit of present invention may be configured such that, for example, the liquid transfer portion is a breaking portion for breaking a bottom surface of the reagent chamber, the breaking portion is disposed so as to be apart from the bottom surface in a direction away from the bottom surface of the reagent chamber, and the first reagent solution in the reagent chamber is transferred to the holding portion when the breaking portion is brought into contact with the bottom surface by force applied from an outside of the reagent chamber, thereby breaking the bottom surface.

In the analysis kit of the present invention, the first binding substance is an aptamer, for example.

In the analysis kit of the present invention, the second binding substance is a nucleic acid molecule complementary to the aptamer, for example.

In the analysis kit of the present invention, the labeling substance may be at least one substance selected from the group consisting of enzymes, nucleic acids, fluorescent substances, dye substances, luminescent substances, radioactive substances, and electron donors, for example.

In the analysis kit of the present invention, the carrier is a bead, for example.

In the analysis kit of the present invention, the second chamber is provided with at least one through hole, an openable closing member is disposed on the through hole in a state of covering the through hole, and liquid transfer from the first chamber to the second chamber is allowed by releasing the through hole from the closing member, for example.

In the analysis kit of the present invention, the closing member is a peelable sealing member, the sealing member is disposed on an outer surface of the second chamber in a state of covering the through hole, and liquid transfer from the first chamber to the second chamber is allowed by peeling off the sealing member from the through hole, for example.

In the analysis kit of the present invention, the closing member is a removable rod-shaped member, the rod-shaped member is disposed on an outer surface of the second chamber in a state of covering the through hole, and liquid transfer from the first chamber to the second chamber is allowed by removing the rod-shaped member from the through hole, for example.

In the analysis kit of the present invention, the first chamber has a front partition wall that is broken upon contact with a tip of the sample holder on a side opposite to the second chamber, for example.

In the analysis kit of the present invention, the first chamber is disposed continuously with a pretreatment chamber with the pretreatment chamber being on a side opposite to the second chamber, the pretreatment chamber is configured such that the sample holder can be inserted from an outside of the pretreatment chamber to an inside of the pretreatment chamber and the pretreatment chamber contains an extractant for extracting a component from the sample, a partition wall is provided between the pretreatment chamber and the first chamber, and the partition wall is a partition wall that is broken upon contact with a tip of the sample holder inserted into the pretreatment chamber, for example.

In the analysis kit of the present invention, the pretreatment chamber includes a cover for an opening of the pretreatment chamber on a side opposite to the partition wall between the pretreatment chamber and the first chamber, and the cover is configured such that the sample holder can be inserted from an outside of the cover to an inside of the pretreatment chamber, for example.

In the analysis method of the present invention, for example, the target analysis kit of the present invention including the tube and the sealing portion is used, and the first reagent solution in the reagent chamber is transferred to the holding portion when the liquid transfer portion is broken inside the reagent chamber by force applied from an outside of the reagent chamber, thereby opening the tube.

In the analysis method of the present invention, for example, the target analysis kit of the present invention including the tube and the lid is used, and the first reagent solution in the reagent chamber is transferred to the holding portion when the lid is moved by force applied from an outside of the reagent chamber, thereby opening the connected portion.

In the analysis method of the present invention, for example, the target analysis kit of the present invention including the breaking portion is used, and the first reagent solution in the reagent chamber is transferred to the holding portion when the breaking portion is brought into contact with the bottom surface by force applied from an outside of the reagent chamber, thereby breaking the bottom surface.

In the present invention, the term "upward direction" means, for example, a direction that is perpendicular to the planar direction of the bottom surface of the analysis container of the present invention, which also is a direction extending from the second chamber toward the first chamber, and the term "downward (bottom) direction" means a direction opposite to the upward direction.

### [Target Analysis Kit and Target Analysis Method]

The target analysis kit of the present invention is, as described above, a target analysis kit including: a sample holder; and an analysis container. The sample holder includes: a reagent chamber containing a first reagent solution that is a solution containing a first reagent; a sample holding portion; and a liquid transfer portion that allows transfer of the first reagent solution in the reagent chamber to the holding portion. The analysis container includes: a first chamber; and a second chamber, The first chamber and the second chamber are disposed continuously in this order. The first chamber contains a second reagent. The second chamber is a detection portion in which a labeling substance contained in the first reagent or the second reagent is to be detected. A porous partition wall is provided between the first chamber and the second chamber. The first chamber is configured such that the sample holder holding a sample can be inserted from an outside of the first chamber to an inside of the first chamber. The porous partition wall is a porous partition wall through which an immobilized first binding substance obtained by immobilizing on a carrier a first binding substance that binds to a target and contained in the first reagent or the second reagent or an immobilized second binding substance obtained by immobilizing on a carrier a second binding substance that binds to the first binding substance and contained in the first reagent or the second reagent cannot pass and a conjugate composed of a labeled first binding substance obtained by binding a labeling substance to the first binding substance and the target and contained in the first reagent or the second reagent or a labeled second binding substance obtained by binding a labeling substance to the second binding substance and contained in the first reagent or the second reagent can pass. A combination of the first reagent and the second reagent is any of the following combinations (1) to (3) and (4):
(1) the first reagent is the immobilized second binding substance and the second reagent is the labeled first binding substance;
(2) the first reagent is the labeled first binding substance and the second reagent is the immobilized second binding substance;
(3) the first reagent is the immobilized first binding substance and the second reagent is the labeled second binding substance; and
(4) the first reagent is the labeled second binding substance and the second reagent is the immobilized first binding substance.

The target analysis method of the present invention is, as described above, a target analysis method using the target analysis kit according to the present invention. The target analysis method includes the steps of: introducing a sample and the first reagent solution to the first chamber by inserting the sample holder holding the sample into the first chamber of the analysis container and then transferring the first reagent solution in the reagent chamber to the holding portion; in the first chamber, bringing the sample into contact with the first reagent and the second reagent to form a conjugate of the target in the sample and the first binding substance and also binding an unbound first binding substance not bound to the target to the second binding substance; introducing an unbound labeled second binding substance not bound to the immobilized first binding substance or an unbound labeled first binding substance not bound to the immobilized second binding substance to the second chamber through the porous partition wall between the first chamber and the second chamber; and detecting the labeling substance in the labeled first binding substance or the labeled second binding substance in the second chamber.

The term "analysis" as used in the present invention may mean, for example, qualitative analysis to determine the presence or absence of a target or quantitative analysis to determine the amount of a target.

In the present invention, the first binding substances that bind to a target to be used are not limited to a particular type of binding substances, as long as they bind to the target, for example. Specifically, the first binding substances may be aptamers or antibodies, for example.

The analysis kit and the analysis method according to the present invention will be described below with reference to drawings and illustrative examples. It is to be noted, however, that the present invention is not limited to or restricted by these illustrative examples. In the drawings, the structure of each component may be shown in an simplified form as appropriate for the sake of convenience in illustration, and each component may be shown schematically with a dimension ratio and the like that are different from the actual dimension ratio and the like. Unless otherwise stated, each of the example embodiments can be combined with each other.

### (First Example embodiment)

The present example embodiment relates to an analysis kit configured such that: the combination of the first reagent and the second reagent is the combination (1); a tube that connects the reagent chamber and the holding portion and a sealing portion that seals the tube in the reagent chamber are included; and the first reagent solution in the reagent chamber is transferred to the holding portion when the liquid transfer portion inside the reagent chamber is broken by force applied from an outside of the reagent chamber, thereby opening the tube, and also relates to an analysis method using this kit. In the present example embodiment, the combination of the first reagent and the second reagent is not limited thereto, and may be the combination (2), (3), or (4), for example, and the descriptions regarding the first reagent and the second reagent should be interpreted as interchangeable with each other.

The analysis kit of the first example embodiment includes: a sample holder; and an analysis container. The sample holder includes: a reagent chamber containing, as a first reagent, a first reagent solution that is a solution containing immobilized second binding substances obtained by immobilizing on a carrier(s) second binding substances that bind to first binding substances that bind to a target; a sample holding portion; and a liquid transfer portion that allows transfer of the first reagent solution in the reagent chamber to the holding portion. The analysis container includes: a first chamber and a second chamber. The first chamber and the second chamber are disposed continuously in this order. The first chamber contains, as a second reagent, labeled first binding substances obtained by binding labeling substances to the first binding substances. The second chamber is a detection portion in which the labeling substances contained in the labeled first binding substances are to be detected. A porous partition wall is provided between the first chamber and the second chamber. The first chamber is configured such that the sample holder holding a sample can be inserted from an outside of the first chamber to an inside of the first chamber. The porous partition wall is a porous partition wall through which the immobilized second binding substances cannot pass and conjugates of the labeled first binding substances with the target can pass. The liquid transfer portion includes: a tube that connects the reagent chamber and the holding portion; and a sealing portion that seals the tube in the reagent chamber, and the first reagent solution in the reagent chamber is transferred to the holding portion when the liquid transfer portion inside the reagent chamber is broken by force applied from an outside of the reagent chamber, thereby opening the tube.

The analysis method of the first example embodiment is an analysis method using the target analysis kit of the first example embodiment, including the steps of: introducing a sample and the first reagent solution to the first chamber by inserting the sample holder holding the sample into the first chamber of the analysis container and then transferring the first reagent solution in the reagent chamber to the holding portion by breaking the liquid transfer portion in the reagent chamber by force applied from an outside of the reagent chamber, thereby opening the tube; in the first chamber, bringing the sample into contact with the first reagent and the second reagent to form conjugates of the target in the sample and labeled first binding substances and also binding unbound labeled first binding substances not bound to the target to immobilized second binding substances; introducing unbound labeled first binding substances not bound to the immobilized second binding substances and the conjugates to the second chamber through the porous partition wall between the first chamber and the second chamber; and detecting the labeling substances in the labeled first binding substances in the second chamber.

According to the present example embodiment, first, in the first chamber, for example, a target in a sample, the immobilized second binding substances as the first reagent, and the labeled first binding substances as the second reagent are mixed together. Then, in the first chamber, the labeled first binding substances as the second reagent bind to the target in the sample, and the labeled first binding substances not bound to the target bind to the immobilized second binding substances as the first reagent. A porous partition wall between the first chamber and the second chamber is the porous partition wall through which the immobilized second binding substances cannot pass and the conjugates of the labeled first binding substances and the target can pass. Accordingly, the immobilized second binding substances do not pass through the porous partition wall and thus remain in the first chamber. That is, the labeled first binding substances bound to the immobilized second binding substances remain in the first chamber without moving to the second chamber. On the other hand, the labeled first binding substances that are in a free state without being bound to the immobilized second binding substances, i.e., the labeled first binding substances forming the conjugates are introduced to the second chamber through the porous partition wall. The analysis kit of the present example embodiment can contain a known amount of the labeled first binding substances, for example. Accordingly, the amount of the labeled first binding substances not bound to the immobilized second binding substances indirectly corresponds to the amount of the target in the sample. Thus, by detecting the unbound labeled first binding substances that are not bound to the immobilized second binding substances and are introduced to the second chamber, the presence or absence of the target or the amount of the target in the sample can be analyzed indirectly.

The first target analysis kit according to the first example embodiment and the target analysis method using the same according to the first example embodiment will be described below with reference to an example where aptamers are used as the first binding substances.

In the present example embodiment, the first binding substances in the labeled first binding substances are aptamers. The aptamers are not limited as long as they can bind to the target. The aptamer may be, for example, a DNA aptamer, an RNA aptamer, or a chimera aptamer containing DNA and RNA. The aptamer may consist of a natural nucleic acid or a non-natural nucleic acid, or may contain a natural nucleic acid and a non-natural nucleic acid. The aptamer may be a modified aptamer, for example. The aptamer may be single-stranded, for example.

The second binding substances in the immobilized second binding substances are not limited as long as they can bind to the aptamers, and may be, for example, nucleic acid molecules complementary to the aptamers (also referred to as "complementary nucleic acid molecules" or "complementary strands" hereinafter). The nucleic acid molecules complementary to the aptamers are not limited to those that are 100% complementary to the aptamers, and may have complementarity sufficient to hybridize to the aptamers or a partial sequence thereof, for example. The complementarity may be, for example, at least 95%, at least 96%, at least 97%, at least 98%, at least 99%, or 100%. By using the complementary nucleic acid molecules as the second binding substances, a reaction among the target, the labeled first binding substances, and the immobilized second binding substances can be caused easily, for example. Thus, the reaction time can be shortened, and also, a higher sensitivity and a broader dynamic range can be achieved. It is preferable that the complementary nucleic acid molecules do not bind to the target, for example.

Examples of the labeling substance in the labeled first binding substance includes enzymes, nucleic acids, fluorescent substances, dye substances, luminescent substances, radioactive substances, and electron donors. The nucleic acid may be a catalytic nucleic acid molecule exhibiting a catalytic function, for example. The enzyme may be, for example, luciferase, peroxidase such as horseradish peroxidase (HRP), or alkaline phosphatase.

The carriers in the immobilized second binding substances may be beads, for example. The material of the beads is not particularly limited, and may be a polymer such as agarose, sepharose, or cellulose, for example. The beads may be magnetic beads, for example. The magnetic beads may be, for example, beads consisting of a magnetic material, beads containing the magnetic material, or beads coated with the magnetic material. The magnetic material may be, for example, a magnetizable substance, and specific examples thereof include γFe₂O₃ and Fe₃O₄. The shape of the beads is not particularly limited, and may be spherical such as completely spherical, for example. The average diameter of the beads is not particularly limited, and may be from 1 to 10 µm, 10 to 100 µm, or 100 to 1000 µm, for example. The carriers may be formed using a resin such as Sepharose or Sephadex, for example. When the carriers are the beads, the amount of the complementary strands to be immobilized on the carriers (beads) is not particularly limited, and may be, for example, from 0.1 fmol to 100 pmol, 1 fmol to 10 pmol, or 10 fmol to 1 pmol per mm² surface area of the bead. In the case where the immobilized first binding substances or the immobilized second binding substances are disposed in the first chamber, the carrier may be an inner wall of the first chamber. When the carrier is the inner wall of the first chamber, the amount of the first binding substances or the second binding substances to be immobilized on the carrier (inner wall) is not particularly limited, and may be, for example, from 0.1 fmol to 100 pmol, 1 fmol to 10 pmol, or 10 fmol to 1 pmol per mm² area of the inner wall of the second chamber.

The first reagent solution is a solution containing the immobilized second binding substances as the first reagent. The immobilized first binding substances are dispersed in a solvent, for example. The solvent may be, for example, an aqueous solvent such as water, a buffer solution, physiological saline, or a mixture thereof. The first reagent solution may further contain an extractant for extracting a component(s) from the sample. The extractant is not particularly limited, and can be selected as appropriate depending on the type of the sample to be subjected to the analysis, the type of a component to be analyzed, etc.

The catalytic nucleic acid molecules are not particularly limited, and may be, for example, a DNAzyme or an RNAzyme. The catalytic nucleic acid molecules in the labeled first binding substances preferably exhibit a catalytic function regardless of whether the target has been bound to the labeled first binding substances, for example. The form of the binding between the binding substance and the catalytic nucleic acid molecule is not particularly limited, and may be phosphodiester bond, for example. The binding substance and the catalytic nucleic acid molecule may be bound together directly or indirectly via a linker, for example. The linker may be a nucleic acid molecule consisting of at least one of DNA and RNA, for example. The catalytic nucleic acid molecule preferably is single-stranded, for example. When the labeling substance is the catalytic nucleic acid molecule, the catalytic nucleic acid molecule may serve both as the labeling substance and the first binding substance. That is, if the catalytic nucleic acid molecule is complementary to the first binding substance, the catalytic nucleic acid molecule can be used as the labeled first binding substance.

The sample is not particularly limited, and may be a food-derived sample, for example. Examples of the food-derived sample include foods, food ingredients, food additives, attached substances in food-processing factories, kitchens, etc., and liquid obtained after washing food-processing factories, kitchens, etc. The form of the sample is not particularly limited, and the sample may be liquid or solid, for example. When the sample is solid, the sample may be in the form of a mixed solution, an extract, a dissolved solution, or the like prepared using a solvent, for example. The solvent is not particularly limited, and may be, for example, water, physiological saline, or a buffer. The sample may or may not contain the target, or whether the sample contains the target may be unknown, for example.

In the present example embodiment, as described above, aptamers can be used as the first binding substances, and complementary nucleic acid molecules can be used as the second binding substances. Thus, the target analysis kit of the present example embodiment is thermostable and can be stored more easily, for example. It is also possible to use an enzyme such as luciferase, alkaline phosphatase, or peroxidase as the labeling substances, for example, and therefore, it is possible to analyze a target with high sensitivity, for example.

The porous partition wall between the first chamber and the second chamber is a porous partition wall through which the immobilized second binding substances cannot pass and the conjugates can pass. The pore size of the porous partition wall can be set as appropriate depending on the sizes of the immobilized second binding substances and the conjugates, for example. The pore size of the porous partition wall is, for example, 0.2 to 100 µm, 0.2 to 50 µm, or 0.5 to 10 µm. In the case where the combination of the first reagent and the second reagent is the combination (3) or (4), i.e., the combination of the immobilized first binding substances and the labeled second binding substances, the porous partition wall may be a porous partition wall through which the immobilized first binding substances cannot pass and the labeled second binding substances can pass. The pore size of the porous partition wall can be set as appropriate depending on the sizes of the immobilized first binding substances and the labeled second binding substances, for example.

When catalytic nucleic acid molecules are used as the labeling substances, it is preferable that the first chamber further contains, as the second reagent, adsorbent carriers that adsorb at least one of proteins and lipids. When the first chamber contains the adsorbent carriers, the adsorbent carriers adsorb proteins, lipids, and the like in the first chamber. This prevents, for example, proteins, lipids, and the like that may affect the catalytic function of the catalytic nucleic acid molecules from being introduced to the second chamber, and therefore, the unbound labeled first binding substances in the second chamber can be detected more accurately. In the present example embodiment, the aptamers as the first binding substances that bind to the target and the complementary nucleic acid molecules as the second binding substances that bind to the first binding substances are both nucleic acids. Thus, by holding the proteins, lipids, etc., which are components other than nucleic acids, in the first chamber using the adsorbent carriers, target analysis can be carried out more accurately.

The material of the adsorbent carriers is not particularly limited, and may be, for example, silica, a crosslinked polymer having a porous structure, and active carbon. The shape of the adsorbent carriers is not particularly limited, and the adsorbent carriers may be beads, for example. Preferably, the adsorbent carriers are silica beads, for example. The size of the adsorbent carriers is not particularly limited, and preferably is such that the adsorbent carriers cannot pass through the porous partition wall, for example. The adsorbent carriers may have the same size as the carriers in the immobilized second binding substances, for example.

When the labeling substances in the labeled first binding substances are the catalytic nucleic acid molecules or the enzyme, it is preferable that the second chamber further contains a substrate for the catalytic function of the catalytic nucleic acid molecules or the enzyme, for example. As the substrate, ATP and luciferin may be used in combination, or a luminol reaction solution may be used, for example.

An example of an analysis method using the analysis kit of the first example embodiment will be described specifically with reference to drawings. It is to be noted, however, that the present invention is not limited to this illustrative example.

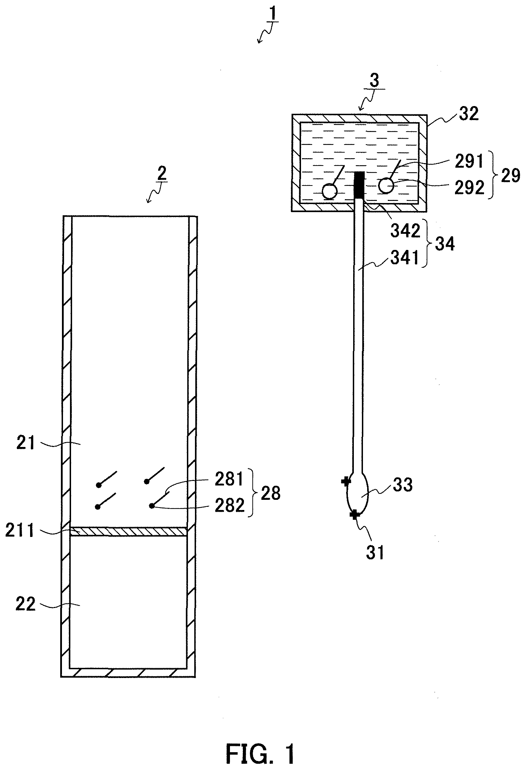

FIG. 1 schematically shows an analysis kit of the first example embodiment. As shown in FIG. 1, an analysis kit 1 of the first example embodiment includes an analysis container 2 and a sample holder 3. The analysis container 2 has a first chamber 21 and a second chamber 22 disposed continuously in this order from the opening side, and has a porous partition wall 211 provided between the first chamber 21 and the second chamber 22. In the first chamber 21, labeled aptamers 28 obtained by adding an enzyme 282 to aptamers 281 are disposed. On the other hand, the sample holder 3 has a reagent chamber 32, a holding portion 33, and a liquid transfer portion 34 connecting the reagent chamber 32 and the holding portion 33. In the reagent chamber 32, a first reagent solution containing immobilized complementary strands 29 obtained by immobilizing complementary strands 291 to the aptamers 281 on beads is disposed. The liquid transfer portion 34 includes a tube 341 and a sealing portion 342 that seals the tube 341 in the reagent chamber 32. An end of the tube 341 on the reagent chamber 32 side is sealed by the sealing portion 342, and the other end of the tube 341 is connected to the holding portion 33. The sample holder 3 collects a sample with the holding portion 33. The target 31 etc. in the sample adhere to the holding portion 33, for example.

The shape of the analysis container 2 of the present example embodiment is not particularly limited, and may be any shape. The material forming the analysis container 2 of the present example embodiment is not particularly limited, and may be any material. Specific examples thereof include plastics such as polyethylene, polystyrene, polypropylene, and acrylonitrile-butadiene-styrene copolymer synthetic resins. In the analysis container 2, the sizes of the first chamber 21 and the second chamber 22 are not particularly limited, and can be designed as appropriate depending on, for example, the volume and the like of the sample to be analyzed.

The porous partition wall 211 between the first chamber 21 and the second chamber 22 is, as described above, a porous partition wall. The porous partition wall is not particularly limited, and may be a porous membrane or the like, for example. The porous membrane may be, for example, a cellulose membrane, a membrane formed of a cellulose derivative such as cellulose acetate or nitrocellulose, a filter such as a glass filter, a filter paper, or the like. The pore size of the porous partition wall is not particularly limited, and can be set as appropriate depending on the reagent etc. to be disposed in the analysis container 2.

In the sample holder 3 of the present example embodiment, the shape of the reagent chamber 32 is not particularly limited, and may be any shape. The material forming the reagent chamber 32 is not particularly limited, and may be, for example, a material that can be deformed by force applied from the outside of the reagent chamber 32. Specific examples thereof include the above-described plastics.

The shape of the holding portion 33 is not particularly limited, and the holding portion 33 may have a structure with which a sample can be collected easily, such as a structure in the form of a swab, in a spoon-like shape, or in a ring shape. The material forming the holding portion 33 is not particularly limited, and may be, for example, a material that can be impregnated with a liquid and has liquid permeability. Specific examples of the material include fibrous materials such as cotton and synthetic fibers; synthetic resins such as foamed urethane; and combinations thereof. The material having liquid permeability means, for example, a material that allows the first reagent solution that has moved inside the holding portion 33 via the tube 341 to move to the outside of the holding portion 33.

The tube 341 is a hollow tube, and when the liquid transfer portion 34 is broken in the reagent chamber 32, transfer of the first reagent solution inside the tube is allowed. Although the tube 341 does not have a bottom in the present example embodiment, there is no limitation thereto, and the tube 341 may be a bottomed tube having a bottom on the lower end side. In this case, the tube 341 has at least one through hole at its end on the holding portion 33 side, for example. Then, in the case where the tube 341 is a bottomed tube with the through hole, when the liquid transfer portion 34 is broken, the first reagent solution moves inside the tube, flows out from the tube via the through hole, and moves along the outer wall of the tube 341. In this manner, the first reagent solution transfers to the holding portion 33. The material forming the tube 341 is not particularly limited, and examples thereof include the above-described plastics. The size of the tube 341 is not particularly limited. For example, the tube 341 may have a length with which, when the reagent holder 3 is disposed in the analysis container 2, the lower end of the holding portion 33 is in contact with the porous partition wall 211 or is not in contact with the porous partition wall 211. Preferably, the length of the tube 341 is the latter.

Although the sealing portion 342 has a rod shape in the present example embodiment, the shape of the sealing portion 342 is not limited thereto as long as it can seal the tube 341, specifically, as long as it can prevent transfer of the first reagent solution in the reagent chamber 32 to the holding portion 33 in the state where the liquid transfer portion 34 is not broken. The material forming the sealing portion 342 is not particularly limited, and examples thereof include the above-described plastics. The size of the sealing portion 342 is not particularly limited. Although the size of the sealing portion 342 is substantially the same as the size of the tube 341 in the present example embodiment, the present invention is not limited thereto, and the size of the sealing portion 342 may be different from the size of the tube 341. The sealing portion 342 may have a shape with which a connected portion with the tube 341 can be broken easily by force applied from the outside of the reagent chamber 32 as compared with other portions of the liquid transfer portion 34, for example.

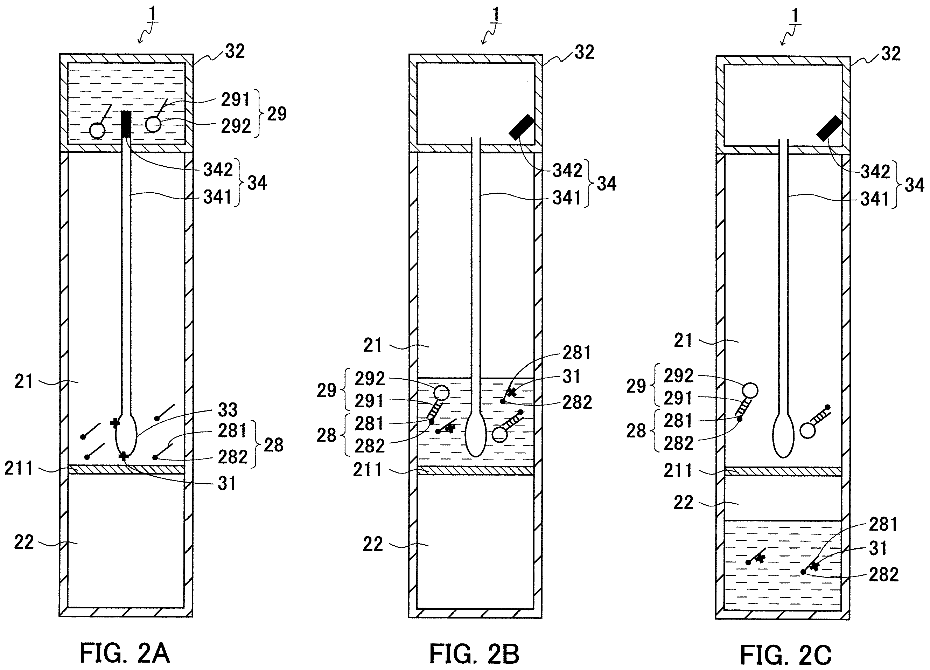

Next, an analysis method using the analysis kit 1 shown in FIG. 1 will be described with reference to FIG. 2. FIG. 2 schematically shows the method of using the analysis kit 1.

First, as shown in FIG. 2A, the sample holder 3 holding a sample in the holding portion 33 is inserted into the first chamber 21 of the analysis container 2 in the analysis kit. Next, by pressing the sample holder 3, the liquid transfer portion 34 is broken in the reagent chamber 32 and the tube 341 is opened. Then, as shown in FIG. 2B, liquid transfer through the tube 341 is allowed, and the first reagent in the reagent chamber 32 moves from the holding portion 33 to the first chamber 21 via the opening and the interior of the tube 341. Then, in the first chamber 21, the target 31 in the sample, the immobilized complementary strands 29, and the labeled aptamers 28 are mixed together. As a result of the mixing, the labeled aptamers 28 form conjugates with the target 31, and the labeled aptamers 28 not bound to the target 31 bind to the immobilized complementary strands 29. Conditions of the treatment for mixing them are not particularly limited, and may be as follows, for example: the treatment temperature is from 4°C to 37°C and the treatment time is from 10 seconds to 30 minutes. The treatment for mixing them preferably is performed in a liquid solvent, for example, and the liquid solvent may be, for example, an aqueous solvent such as water, a buffer solution, physiological saline, or a mixture thereof.

The porous partition wall 211 is a porous partition wall through which the immobilized complementary strands 29 do not pass and the conjugates of the labeled aptamers 28 with the target pass. Accordingly, among the labeled aptamers 28, only those not bound to the immobilized complementary strands 29 pass through the porous partition wall 211 to be introduced into the second chamber 22. Then, in the second chamber 22, by subjecting the labeled aptamers 28 not bound to the immobilized complementary strands 29 to measurement of the catalytic function of the enzyme 282, the target in the sample can be analyzed indirectly. The method for measuring the catalytic function of the enzyme 282 can be determined as appropriate depending on the type of the enzyme 282.

The analysis container 2 of the present example embodiment may further include one or more of the following example embodiments. The following respective example embodiments can be combined with the analysis containers of the second and third example embodiments to be described below.

In the analysis container 2, the second chamber 22 may be provided with at least one through hole. In this case, an openable closing member is disposed on the through hole in a state of covering the through hole, and liquid transfer from the first chamber 21 to the second chamber 22 is allowed by releasing the through hole from the closing member. The closing member may be, for example, a peelable sealing member, a removable rod-shaped member, or the like. When the analysis container 2 includes, for example, the through hole and the closing member, opening and closing of the through hole can be controlled by the closing member, whereby liquid transfer from the first chamber 21 to the second chamber 22 can be controlled easily.

When the analysis container 2 includes a peelable sealing member as the closing member, in the analysis container 2, for example, the sealing member is disposed on the outer surface of the second chamber 22 in a state of covering the through hole, and by peeling off the sealing member from the through hole, liquid transfer from the first chamber 21 to the second chamber 22 is allowed. The sealing member may be disposed, for example, in a state of covering the through hole or in a state blocking an inside of the through hole. The sealing member is not particularly limited, and may be formed of any material, for example. For example, it is possible to use an adhesive tape, a pressure-sensitive adhesive tape, or the like. The method for disposing the sealing member is not limited as long as the sealing member can be peeled off, and may be adhesion or the like, for example. The sealing member may be adapted such that, for example, after it is peeled off, it can close the through hole again.

When the analysis container 2 includes a removable rod-shaped portion as the closing member, the rod-shaped member is disposed on an outer surface of the second chamber 22 in a state of covering the through hole, and by removing the rod-shaped member from the through hole, liquid transfer from the first chamber 21 to the second chamber 22 is allowed. The rod-shaped member may be disposed on the outer surface of the second chamber 22 in a state of covering the through hole or may be disposed in a state of blocking the through hole. The rod-shaped member is not particularly limited, and may be formed of any material, for example. For example, the rod-shaped member may be a rod-shaped plastic or the like. The material of the rod-shaped member may be the same as the material of the analysis container 2 or may be different from the material of the analysis container 2, for example. The method of disposing the rod-shaped member may be any known method by which the rod-shaped member is disposed in a removable manner, and may be heat-sealing or the like, for example. The rod-shaped member may be adapted such that, for example, after it is removed, it can close the through hole again.

The analysis container 2 may further include a pretreatment chamber, for example. In the analysis container 2, the first chamber 21 may be disposed continuously with the pretreatment chamber with the pretreatment chamber being on a side opposite to the second chamber 22. That is, in the analysis container 2, the pretreatment chamber, the first chamber 21, and the second chamber 22 may be disposed in this order.

The pretreatment chamber is configured such that the sample holder 3 can be inserted from an outside of the pretreatment chamber to an inside of the pretreatment chamber. The pretreatment chamber contains an extractant for extracting a component(s) from the sample, for example. The extractant is not particularly limited, and can be selected as appropriate depending on the type of the sample to be subjected to the analysis, the type of the component(s) to be analyzed, etc.

The analysis container 2 has a partition wall between the pretreatment chamber and the first chamber 21, for example, and the material, the properties, and the like of the partition wall are not particularly limited as long as the partition wall can be broken upon contact with the tip of the sample holder 3 inserted into the pretreatment chamber. The partition wall can be formed of a thin metal film (such as aluminum foil), paper, or synthetic fibers, for example.

The analysis container 2 may be configured such that, for example, the pretreatment chamber has a cover for an opening of the pretreatment chamber on a side opposite to the partition wall between the pretreatment chamber and the first chamber 21, and the sample holder 3 can be inserted from an outside of the cover to an inside of the pretreatment chamber. The cover preferably is a partition wall that is broken upon contact with the tip of the sample holder, and may be the same as the above-described partition wall, for example.

Furthermore, in the analysis container 2, the first chamber may further include, for example, a front partition wall that is broken upon contact with the tip of the sample holder on a side opposite to the second chamber. The above description regarding the partition wall of the pretreatment chamber also applies to the front partition wall, for example.

### (Second Example embodiment)

The present example embodiment relates to an analysis kit configured such that: the combination of the first reagent and the second reagent is the combination (3); a tube that connects the reagent chamber and the holding portion and a lid disposed so as to block a connected portion between the reagent chamber and the tube are included; and the first reagent solution in the reagent chamber is transferred to the holding portion when the lid is moved by force applied from an outside of the reagent chamber, thereby opening the connected portion, and also relates to an analysis method using this kit. In the present example embodiment, the combination of the first reagent and the second reagent is not limited thereto, and may be the combination of (1), (2), or (4), for example, and the descriptions regarding the first reagent and the second reagent should be interpreted as interchangeable with each other. The above descriptions regarding the analysis kit and analysis method of the first example embodiment also apply to the analysis kit and analysis method of the second example embodiment.

The analysis kit of the second example embodiment includes: a sample holder; and an analysis container. The sample holder includes: a reagent chamber containing, as a first reagent, a first reagent solution that is a solution containing immobilized first binding substances obtained by immobilizing first binding substances that bind to a target on a carrier(s); a sample holding portion; a liquid transfer portion that allows transfer of the first reagent solution in the reagent chamber to the holding portion. The analysis container includes: a first chamber and a second chamber. The first chamber and the second chamber are disposed continuously in this order. The first chamber contains, as a second reagent, labeled second binding substances obtained by binding labeling substances to second binding substances that bind to the first binding substances. The second chamber is a detection portion in which the labeling substances contained in the labeled second binding substances is to be detected. A porous partition wall is provided between the first chamber and the second chamber. The first chamber is configured such that a sample holder holding a sample can be inserted from an outside of the first chamber to an inside of the first chamber. The porous partition wall is a porous partition wall through which the immobilized first binding substances cannot pass and the labeled second binding substances can pass. The liquid transfer portion includes: a tube that connects the reagent chamber and the holding portion; and a lid disposed so as to block a connected portion between the reagent chamber and the tube, and the first reagent solution in the reagent chamber is transferred to the holding portion when the lid is moved by force applied from an outside of the reagent chamber, thereby opening the connected portion.

The analysis method of the second example embodiment is an analysis method using the target analysis kit of the second example embodiment, including the steps of: introducing a sample and the first reagent solution to the first chamber by inserting the sample holder holding the sample into the first chamber of the analysis container and then transferring the first reagent solution in the reagent chamber to the holding portion by moving the lid by force applied from an outside of the reagent chamber, thereby opening the connected portion; in the first chamber, bringing the sample into contact with the first reagent and the second reagent to form conjugates of the target in the sample and immobilized first binding substances and also binding unbound immobilized first binding substances not bound to the target to the labeled second binding substances; introducing unbound labeled second binding substances not bound to the immobilized first binding substances to the second chamber through the porous partition wall between the first chamber and the second chamber; and detecting the labeling substances in the labeled second binding substances in the second chamber.

According to the present example embodiment, first, in the first chamber, for example, a target in a sample is mixed with the immobilized first binding substances as the first reagent and the labeled second binding substances as the second reagent. Then, in the first chamber, the immobilized first binding substances as the first reagent bind to the target in the sample, and the immobilized first binding substances not bound to the target bind to the labeled second binding substances as the second reagent. The porous partition wall between the first chamber and the second chamber is a porous partition wall through which the immobilized first binding substances cannot pass and the labeled second binding substances can pass. Accordingly, the immobilized first binding substances do not pass through the partition wall and thus remain in the first chamber. That is, the labeled second binding substances bound to the immobilized first binding substances remain in the first chamber without moving to the second chamber. On the other hand, the labeled second binding substances that are in a free state without being bound to the immobilized first binding substances are introduced to the second chamber through the porous partition wall. The target analysis kit of the present invention can contain a known amount of the labeled second binding substances, for example. Accordingly, the amount of the labeled second binding substances not bound to the immobilized first binding substances indirectly corresponds to the amount of the target in the sample. Thus, by detecting the unbound labeled second binding substances introduced to the second chamber, the presence or absence of the target or the amount of the target in the sample can be analyzed indirectly.

The analysis kit according to the second example embodiment and the analysis method using the same according to the second example embodiment will be described below with reference to an example where aptamers are used as the first binding substances. Unless otherwise stated, the above descriptions regarding the first example embodiment also apply to the second example embodiment.

An example of the analysis method using the analysis kit of the second example embodiment will be described specifically with reference to drawings. It is to be noted, however, that the present invention is not limited to this illustrative example.

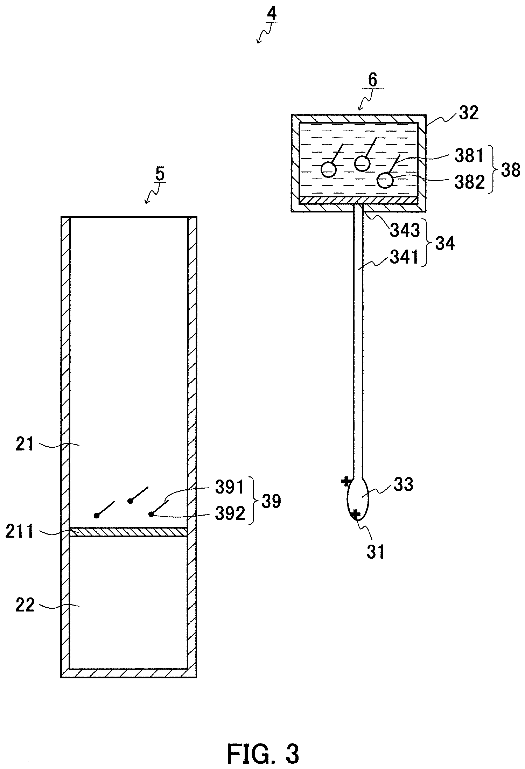

FIG. 3 schematically shows an analysis kit of the present example embodiment. As shown in FIG. 3, an analysis kit 4 of the second example embodiment includes an analysis container 5 and a sample holder 6. The analysis container 5 has a first chamber 21 and a second chamber 22 disposed continuously in this order from the opening side, and has a porous partition wall 211 provided between the first chamber 21 and the second chamber 22. In the first chamber 21, labeled complementary strands 39 obtained by adding an enzyme 392 to complementary strands 391 to aptamers 381 are disposed. On the other hand, the sample holder 6 has a reagent chamber 32, a holding portion 33, and a liquid transfer portion 34 connecting the reagent chamber 32 and the holding portion 33. In the reagent chamber 32, a first reagent solution containing immobilized aptamers 38 obtained by immobilizing aptamers 381 on beads 382 is disposed. The liquid transfer portion 34 includes a tube 341 and a lid 343 disposed so as to block a connected portion between the reagent chamber 32 and the tube 341. An end of the tube 341 on the reagent chamber 32 side is closed by the lid 343, and the other end of the tube 341 is connected to the holding portion 33. The sample holder 6 collects a sample with the holding portion 33. A target 31 and the like in the sample adhere to the holding portion 33, for example.

Although the lid 343 is in a plate-like shape in the present example embodiment, the shape of the lid 343 is not limited thereto as long as it can close the tube 341. More specifically, the lid 343 may have any shape as long as it can prevent transfer of the first reagent solution in the reagent chamber 32 to the holding portion 33 in the state where the liquid transfer portion 34 is not broken. Although the lid 343 covers the opening at the end of the tube 341 on the reagent chamber 32 side in the present example embodiment, the lid 343 may further block an interior of the tube 341. The material forming the lid 343 is not particularly limited, and examples thereof include the above-described plastics. The size of the lid 343 is not particularly limited. In the present example embodiment, the sizes of the lid 343 other than the size in the vertical direction, i.e., the length and the width are substantially the same as the inside dimensions of the bottom of the reagent chamber 32. However, the present invention is not limited thereto, and they may be different from the inside dimensions of the reagent chamber 32. The sample holder 6 of the present example embodiment may include, for example, a fixing member for fixing the lid 343 so as to prevent the lid 343 that has moved by force applied from the outside of the reagent chamber 32 from covering the opening at the end on the reagent chamber 32 side again.

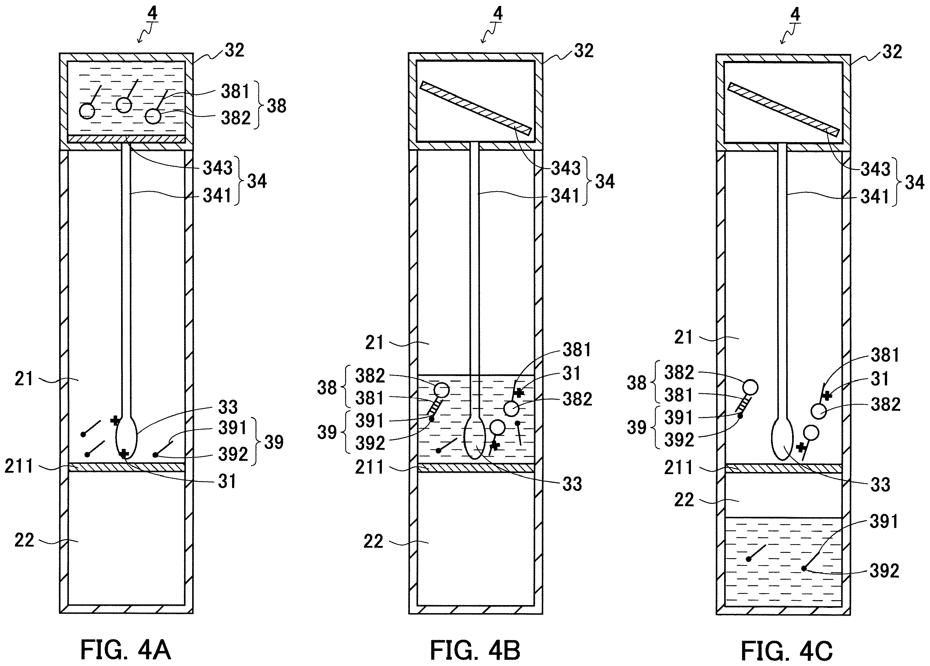

Next, an analysis method using the analysis kit 4 shown in FIG. 3 will be described with reference to FIGs. 4A to 4C. FIGs. 4A to 4C schematically show the method of using the analysis kit 4.

First, as shown in FIG. 4A, the sample holder 6 holding a sample in the holding portion 33 is inserted into the first chamber 21 of the analysis container 5 of the analysis kit. Next, by pressing the sample holder 6, the lid 343 is moved inside the reagent chamber 32, and the tube 341 is opened (released). Then, as shown in FIG. 4B, liquid transfer in the tube 341 is allowed, and the first reagent in the reagent chamber 32 moves from the holding portion 33 to the first chamber 21 via the opening and the interior of the tube 341. Then, in the first chamber 21, the target 31 in the sample, the immobilized aptamers 38, and the labeled complementary strands 39 are mixed together. As a result of the mixing, the target 31 and the immobilized aptamers 38 form conjugates, and the immobilized aptamers 38 not bound to the target 31 bind to labeled complementary strands 39. Conditions of the treatment for mixing them are not particularly limited, and may be as follows, for example: the treatment temperature is from 4°C to 37°C and the treatment time is from 10 seconds to 30 minutes. The treatment for mixing them preferably is performed in a liquid solvent, for example, and the liquid solvent may be, for example, an aqueous solvent such as water, a buffer solution, physiological saline, or a mixture thereof.

The porous partition wall 211 is a porous partition wall through which the immobilized aptamers 38 do not pass and the labeled complementary strands 39 pass. Accordingly, among the labeled complementary strands 39, only those not bound to the immobilized aptamers 38 pass through the second partition wall 211 to be introduced to the second chamber 22. Then, in the second chamber 22, by subjecting the labeled complementary strands 39 not bound to the immobilized aptamers 38 to measurement of the catalytic function of the enzyme 392, the target in the sample can be analyzed indirectly. The method for measuring the catalytic function of the enzyme 392 can be determined as appropriate depending on the type of the enzyme 392.

### (Third Example embodiment)

The present example embodiment relates to an analysis kit configured such that: the combination of the first reagent and the second reagent is the combination (1); the liquid transfer portion is a breaking portion for breaking a bottom surface of the reagent chamber; the breaking portion is disposed so as to be apart from the bottom surface in the direction away from the bottom surface of the reagent chamber, and the first reagent solution in the reagent chamber is transferred to the holding portion when the breaking portion is brought into contact with the bottom surface by force applied from an outside of the reagent chamber, thereby breaking the bottom surface, and also relates to an analysis method using this kit. In the present example embodiment, the combination of the first reagent and the second reagent is not limited thereto, and may be the combination of (2), (3), or (4), for example, and the descriptions regarding the first reagent and the second reagent should be interpreted as interchangeable with each other. The above descriptions regarding the analysis kit and analysis method of the first example embodiment also apply to the analysis kit and analysis method of the second example embodiment.

The analysis kit of the third example embodiment includes: a sample holder; and an analysis container. The sample holder includes: a reagent chamber containing, as a first reagent, a first reagent solution that is a solution containing immobilized second binding substances obtained by immobilizing second binding substances that binds to first binding substances that bind to a target on a carrier(s); a sample holding portion; and a liquid transfer portion that allows transfer of the first reagent solution in the reagent chamber to the holding portion. The analysis container includes: a first chamber and a second chamber. The first chamber and the second chamber are disposed continuously in this order. The first chamber contains, as a second reagent, labeled first binding substances obtained by binding labeling substances to the first binding substances. The second chamber is a detection portion in which the labeling substances contained in the labeled first binding substances are to be detected. A porous partition wall is provided between the first chamber and the second chamber. The first chamber is configured such that a sample holder holding a sample can be inserted from an outside of the first chamber to an inside of the first chamber. The porous partition wall is a porous partition wall through which the immobilized second binding substances cannot pass and conjugates of the labeled first binding substances with the target can pass. The liquid transfer portion is a breaking portion for breaking a bottom surface of the reagent chamber. The breaking portion is disposed so as to be apart from the bottom surface in the direction away from the bottom surface of the reagent chamber. The first reagent solution in the reagent chamber is transferred to the holding portion when the breaking portion is brought into contact with the bottom surface by force applied from an outside of the reagent chamber, thereby breaking the bottom surface.

The analysis method of the third example embodiment is an analysis method using the target analysis kit of the third example embodiment, including the steps of: introducing a sample and the first reagent solution to the first chamber by inserting the sample holder holding the sample into the first chamber of the analysis container and then transferring the first reagent solution in the reagent chamber to the holding portion by bringing the breaking portion into contact with the bottom surface by force applied from an outside of the reagent chamber, thereby breaking the bottom surface; in the first chamber, bringing the sample into contact with the first reagent and the second reagent to form conjugates of the target in the sample and labeled first binding substances and also binding unbound labeled first binding substances not bound to the target to immobilized second binding substances; introducing unbound labeled first binding substances not bound to the immobilized second binding substances and the conjugates to the second chamber through the porous partition wall between the first chamber and the second chamber; and detecting the labeling substances in the labeled first binding substances in the second chamber.

According to the present example embodiment, first, in the first chamber, for example, a target in a sample, the immobilized first binding substances as the first reagent, and the labeled first binding substances as the second reagent are mixed together. Then, in the first chamber, the labeled first binding substances as the second reagent bind to the target in the sample, and the labeled first binding substances not bound to the target bind to the immobilized second binding substance as the first reagent. A porous partition wall between the first chamber and the second chamber is the porous partition wall through which the immobilized second binding substances cannot pass and the conjugates of the labeled first binding substances and the target can pass. Accordingly, the immobilized second binding substances do not pass through the porous partition wall and thus remain in the first chamber. That is, the labeled first binding substances bound to the immobilized second binding substances remain in the first chamber without moving to the second chamber. On the other hand, the labeled first binding substances that are in a free state without being bound to the immobilized second binding substances, i.e., the labeled first binding substances forming the conjugates are introduced to the second chamber through the porous partition wall. The analysis kit of the present example embodiment can contain a known amount of the labeled first binding substances, for example. Accordingly, the amount of the labeled first binding substances not bound to the immobilized second binding substances indirectly corresponds to the amount of the target in the sample. Thus, by detecting the unbound labeled first binding substances that are not bound to the immobilized second binding substances and are introduced to the second chamber, the presence or absence of the target or the amount of the target in the sample can be analyzed indirectly.

The analysis kit according to the third example embodiment and the analysis method using the same according to the third example embodiment will be described below with reference to an example where aptamers are used as the first binding substances. Unless otherwise stated, the above descriptions regarding the first and second example embodiments also apply to the third example embodiment.

An example of an analysis method using the analysis kit of the third example embodiment will be described specifically with reference to drawings. It is to be noted, however, that the present invention is not limited to this illustrative example.

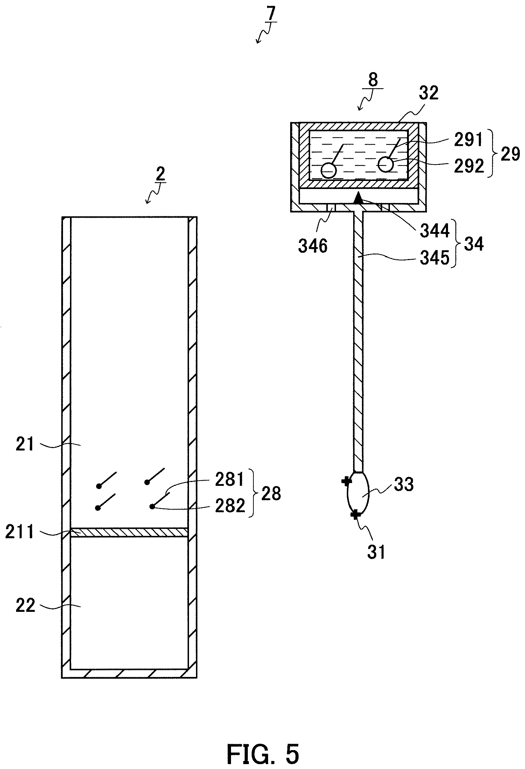

FIG. 5 schematically shows an analysis kit of the present example embodiment. As shown in FIG. 5, an analysis kit 7 of the third example embodiment includes an analysis container 2 and a sample holder 8. The analysis container 2 has a first chamber 21 and a second chamber 22 disposed continuously in this order from the opening side, and has a porous partition wall 211 between the first chamber 21 and the second chamber 22. In the first chamber 21, labeled aptamers 28 obtained by adding an enzyme 282 to aptamers 281 are disposed. On the other hand, the sample holder 8 has a reagent chamber 32, a holding portion 33, and a liquid transfer portion 34 connecting the reagent chamber 32 and the holding portion 33. In the reagent chamber 32, a first reagent solution containing immobilized complementary strands 29 obtained by immobilizing complementary strands 291 to aptamers 281 on beads 292 is disposed. The liquid transfer portion 34 has a breaking portion 344 and a supporting portion 345. The supporting portion 345 has a bottomed tube and a rod, the bottomed tube is disposed on an upper end of the rod, and the holding portion 33 is disposed on a lower end of the rod. The reagent portion 32 is disposed in an upper part of the bottomed tube so as to be in contact with an inner surface of the bottomed tube. The breaking portion 344 is disposed on a bottom surface of the bottomed tube in a state of being apart from the bottom surface of the reagent chamber 32 in the direction away from the bottom surface of the reagent chamber 32. The bottom surface of the bottomed tube is provided with through holes 346 through which the first reagent solution can pass. The sample holder 8 collects a sample with the holding portion 33. The target 31 and the like in the sample adhere to the holding portion 33, for example. The supporting portion 345 is an optional component, and may or may not be provided.

In the present example embodiment, the bottom surface of the reagent chamber 32 may be formed of a material that can be broken by the breaking portion 344, and the material may be, for example, a material that is deformable and breakable.

Although the breaking portion 344 is a member with a shape tapered upward in the present example embodiment, the shape of the breaking portion 344 is not limited thereto as long as the breaking portion 344 can break the bottom surface of the reagent chamber 32. The material forming the breaking portion 344 is not particularly limited, and can be determined as appropriate depending on the material forming the bottom surface of the reagent chamber 32, for example. The size of the breaking portion 344 is not particularly limited. Although only one breaking portion 344 is provided in the present example embodiment, two or more breaking portions 344 may be provided.

The size and the shape of the through hole 346 are not particularly limited, and for example, the through hole 346 may have a size and a shape that allow the first reagent solution containing the immobilized complementary strands 29 to pass through the through hole 346.

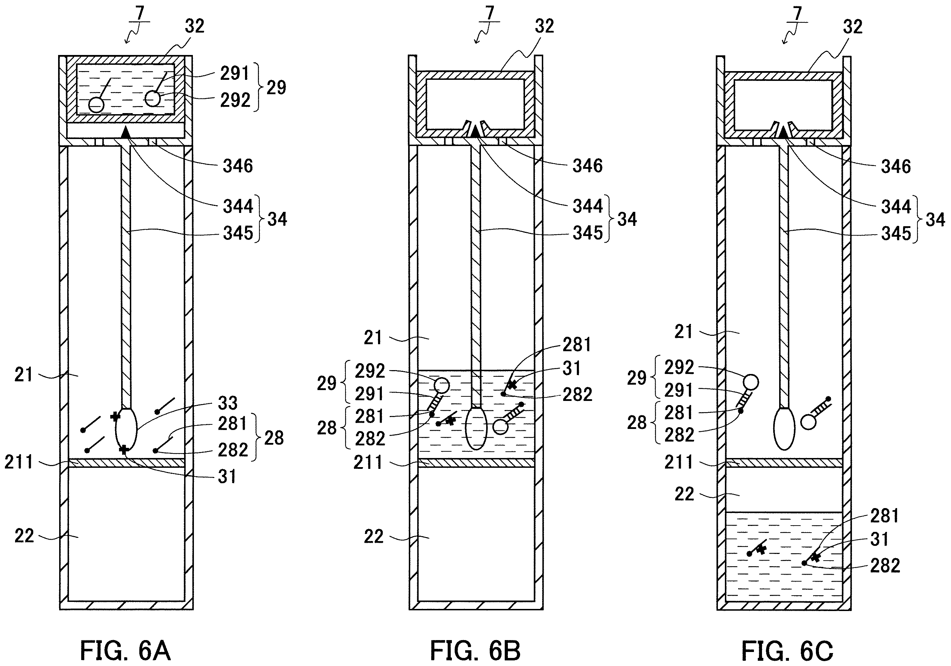

Next, an analysis method using the analysis kit 7 shown in FIG. 5 will be described with reference to FIGs. 6A to 6C. FIGs. 6A to 6C schematically show the method of using the analysis kit 7.

First, as shown in FIG. 6A, the sample holder 8 holding a sample in the holding portion 33 is inserted into the first chamber 21 of the analysis container 2 in the analysis kit. Next, by pressing the sample holder 8 from above, the reagent chamber 32 is moved toward the bottom surface, and the breaking portion 344 is brought into contact with the bottom surface of the reagent chamber 32, whereby the bottom surface of the reagent chamber 32 is broken. Then, as shown in FIG. 6B, liquid transfer from the reagent chamber 32 to the holding portion 33 is allowed, and the first reagent in the reagent chamber 32 moves from the holding portion 33 to the first chamber 21 via an opening formed by the breakage on the bottom surface of the reagent chamber 32 and the supporting portion 345. Then, in the first chamber 21, the target 31 in the sample, the immobilized complementary strands 29, and the labeled aptamers 28 are mixed together. Then, as a result of the mixing, the target 31 and labeled aptamers 28 form conjugates, and the labeled aptamers 28 not bound to the target 31 bind to the immobilized complementary strands 29. Conditions of the treatment for mixing them are not particularly limited, and may be as follows, for example: the treatment temperature is from 4°C to 37°C and the treatment time is from 10 seconds to 30 minutes. The treatment for mixing them preferably is performed in a liquid solvent, for example, and the liquid solvent may be, for example, an aqueous solvent such as water, a buffer solution, physiological saline, or a mixture thereof.

The porous partition wall 211 is a porous partition wall through which the immobilized complementary strands 29 do not pass and the conjugates of the labeled aptamers 28 with the target pass. Accordingly, among the labeled aptamers 28, only those not bound to the immobilized complementary strands 29 pass through the porous partition wall 211 to be introduced to the second chamber 22. Then, in the second chamber 22, by subjecting the labeled aptamers 28 not bound to the immobilized complementary strands 29 to measurement of the catalytic function of the enzyme 282, the target in the sample can be analyzed indirectly. The method for measuring the catalytic function of the enzyme 282 can be determined as appropriate depending on the type of the enzyme 282.

This application is based upon and claims the benefit of priority from Japanese Patent Application No. 2016-142546 filed on July 20, 2016, the disclosure of which is incorporated herein its entirety by reference.

Although the present invention has been described above with reference to exemplary example embodiments, the present invention is by no means limited thereto. Various changes and modifications that may become apparent to those skilled in the art may be made in the configuration and specifics of the present invention without departing from the scope of the present invention.

### Industrial Applicability

According to the present invention, a target in a sample can be analyzed easily.

### Reference Signs List

- 1, 4, 7:: target analysis kit
- 2, 5:: analysis container
- 21:: first chamber
- 22:: second chamber
- 211:: porous partition wall
- 28:: labeled aptamer
- 281, 381:: aptamer
- 282, 392:: enzyme
- 29:: immobilized complementary strand
- 291, 391:: complementary strand
- 292, 382:: bead
- 3, 6, 8:: sample holder
- 31:: target
- 32:: reagent chamber
- 33:: holding portion
- 34:: liquid transfer portion
- 341:: tube
- 342:: sealing portion
- 343:: lid
- 344:: breaking portion
- 345:: supporting portion
- 346:: through hole
- 38:: immobilized aptamers
- 39:: labeled complementary strand

## Claims

1. A target analysis kit comprising:
a sample holder; and
an analysis container,
the sample holder comprising:
a reagent chamber containing a first reagent solution that is a solution containing a first reagent;
a sample holding portion; and
a liquid transfer portion that allows transfer of the first reagent solution in the reagent chamber to the holding portion,
the analysis container comprising:
a first chamber; and
a second chamber,
wherein the first chamber and the second chamber are disposed continuously in this order,
the first chamber contains a second reagent,
the second chamber is a detection portion in which a labeling substance contained in the first reagent or the second reagent is to be detected,
a porous partition wall is provided between the first chamber and the second chamber,
the first chamber is configured such that the sample holder holding a sample can be inserted from an outside of the first chamber to an inside of the first chamber,
the porous partition wall is a porous partition wall through which an immobilized first binding substance obtained by immobilizing on a carrier a first binding substance that binds to a target and contained in the first reagent or the second reagent or an immobilized second binding substance obtained by immobilizing on a carrier a second binding substance that binds to the first binding substance and contained in the first reagent or the second reagent cannot pass and a conjugate composed of a labeled first binding substance obtained by binding a labeling substance to the first binding substance and the target and contained in the first reagent or the second reagent or a labeled second binding substance obtained by binding a labeling substance to the second binding substance and contained in the first reagent or the second reagent can pass, and
a combination of the first reagent and the second reagent is any of the following combinations (1) to (3) and (4):
(1) the first reagent is the immobilized second binding substance and the second reagent is the labeled first binding substance;
(2) the first reagent is the labeled first binding substance and the second reagent is the immobilized second binding substance;
(3) the first reagent is the immobilized first binding substance and the second reagent is the labeled second binding substance; and
(4) the first reagent is the labeled second binding substance and the second reagent is the immobilized first binding substance.

2. The target analysis kit according to claim 1, wherein
the liquid transfer portion comprises: a tube that connects the reagent chamber and the holding portion; and a sealing portion that seals the tube in the reagent chamber, and
the first reagent solution in the reagent chamber is transferred to the holding portion when the liquid transfer portion is broken inside the reagent chamber by force applied from an outside of the reagent chamber, thereby opening the tube.

3. The target analysis kit according to claim 1, wherein
the liquid transfer portion comprises a tube that connects the reagent chamber and the holding portion; and a lid disposed so as to block a connected portion between the reagent chamber and the tube, and
the first reagent solution in the reagent chamber is transferred to the holding portion when the lid is moved by force applied from an outside of the reagent chamber, thereby opening the connected portion.

4. The target analysis kit according to claim 1, wherein
the liquid transfer portion is a breaking portion for breaking a bottom surface of the reagent chamber,
the breaking portion is disposed so as to be apart from the bottom surface in a direction away from the bottom surface of the reagent chamber, and
the first reagent solution in the reagent chamber is transferred to the holding portion when the breaking portion is brought into contact with the bottom surface by force applied from an outside of the reagent chamber, thereby breaking the bottom surface.

5. The target analysis kit according to any one of claims 1 to 4, wherein
the first binding substance is an aptamer.

6. The target analysis kit according to any one of claims 1 to 5, wherein
the second binding substance is a nucleic acid molecule complementary to the aptamer.

7. The target analysis kit according to any one of claims 1 to 6, wherein
the labeling substance is at least one substance selected from the group consisting of enzymes, nucleic acids, fluorescent substances, dye substances, luminescent substances, radioactive substances, and electron donors.

8. The target analysis kit according to any one of claim 1 to 7, wherein the carrier is a bead.

9. The target analysis kit according to any one of claims 1 to 8, wherein
the second chamber is provided with at least one through hole,
an openable closing member is disposed on the through hole in a state of covering the through hole, and
liquid transfer from the first chamber to the second chamber is allowed by releasing the through hole from the closing member.

10. The target analysis kit according to claim 9, wherein
the closing member is a peelable sealing member,
the sealing member is disposed on an outer surface of the second chamber in a state of covering the through hole, and
liquid transfer from the first chamber to the second chamber is allowed by peeling off the sealing member from the through hole.

11. The target analysis kit according to claim 9, wherein
the closing member is a removable rod-shaped member,
the rod-shaped member is disposed on an outer surface of the second chamber in a state of covering the through hole, and
liquid transfer from the first chamber to the second chamber is allowed by removing the rod-shaped member from the through hole.

12. The target analysis kit according to any one of claims 1 to 11, wherein
the first chamber has a front partition wall that is broken upon contact with a tip of the sample holder on a side opposite to the second chamber.

13. The target analysis kit according to any one of claims 1 to 12, wherein
the first chamber is disposed continuously with a pretreatment chamber with the pretreatment chamber being on a side opposite to the second chamber,
the pretreatment chamber is configured such that the sample holder can be inserted from an outside of the pretreatment chamber to an inside of the pretreatment chamber and the pretreatment chamber contains an extractant for extracting a component from the sample,
a partition wall is provided between the pretreatment chamber and the first chamber, and
the partition wall is a partition wall that is broken upon contact with a tip of the sample holder inserted into the pretreatment chamber.

14. The target analysis kit according to claim 13, wherein
the pretreatment chamber has a cover for an opening of the pretreatment chamber on a side opposite to the partition wall between the pretreatment chamber and the first chamber, and
the cover is configured such that the sample holder can be inserted from an outside of the cover to an inside of the pretreatment chamber.

15. A target analysis method using the target analysis kit according to any one of claims 1 to 14, the target analysis method comprising the steps of:
introducing a sample and the first reagent solution to the first chamber by inserting the sample holder holding the sample into the first chamber of the analysis container and then transferring the first reagent solution in the reagent chamber to the holding portion;
in the first chamber, bringing the sample into contact with the first reagent and the second reagent to form a conjugate of the target in the sample and the first binding substance and also binding an unbound first binding substance not bound to the target to the second binding substance;
introducing an unbound labeled second binding substance not bound to the immobilized first binding substance or an unbound labeled first binding substance not bound to the immobilized second binding substance to the second chamber through the porous partition wall between the first chamber and the second chamber; and
detecting the labeling substance in the labeled first binding substance or the labeled second binding substance in the second chamber.

16. The target analysis method according to claim 15, wherein
the target analysis kit according to claim 2 is used, and
the first reagent solution in the reagent chamber is transferred to the holding portion when the liquid transfer portion is broken inside the reagent chamber by force applied from an outside of the reagent chamber, thereby opening the tube.

17. The target analysis method according to claim 15, wherein
the target analysis kit according to claim 3 is used, and
the first reagent solution in the reagent chamber is transferred to the holding portion when the lid is moved by force applied from an outside of the reagent chamber, thereby opening the connected portion.

18. The target analysis method according to claim 15, wherein
the target analysis kit according to claim 4 is used, and
the first reagent solution in the reagent chamber is transferred to the holding portion when the breaking portion is brought into contact with the bottom surface by force applied from an outside of the reagent chamber, thereby breaking the bottom surface.
